# EUROPEAN PATENT APPLICATION

(11) **EP 0 979 650 A1**
(43) Date of publication of application: **16.02.2000**
(21) Application number: 98901036.8
(22) Date of filing: 29.01.1998
(51) Int. Cl.: A61K 31/505, A61K 31/52, A61K 31/675, A61K 31/70

(54) **ANTIVIRAL AGENTS**

(30) Priority: 31.01.1997 JP 1838497
(71) Applicant: MITSUBISHI CHEMICAL CORPORATION, Chiyoda-ku, Tokyo 100-0005 (JP)
(72) Inventor: YUASA, Satoshi, Mitsubishi Chemical Corporation, Yokohama-shi, Kanagawa 227 (JP)
(74) Representative: Godemeyer, Thomas, Dr.
(86) International application number: JP9800361
(87) International publication number: WO9833505

(57) **Abstract**

An antiviral agent against retroviruses exhibiting synergistic antiviral effect which comprises as active ingredients a 6-benzyl-1-ethoxy-methyl-5-substituted uracil derivative such as 6-benzyl-1-ethoxymethyl-5-isopropyluraci together with two or more substances selected from the group consisting of nucleoside-type reverse transcriptase inhibitors such as 3'-azido-3'-deoxythymidine and 2'-deoxy-3'-thiacytidine and esters thereof.

## Description

### Field of the Invention

The present invention relates to antiviral agents. More specifically, the present invention relates to antiviral agents which comprise three or more compounds known to have antiviral activity as active ingredients and can exhibit synergistic effect.

### Background Art

In recent years, infection caused by human acquired immunodeficiency virus (HIV), a kind of retrovirus, has become a big social problem because of the severity of symptoms and high mortality rate. Recently, it has been recognized that a lower viral amount in blood can achieve a higher survival rate of HIV infected patients. Therefore, reduction of the amount of viruses *in vivo* as low as possible is a purpose of antiviral agents in therapeutic treatment of a patient with HIV infection. Various therapies using several drugs simultaneously have been currently studied to obtain higher efficacy of antiviral agents.

Eleven drugs are available at present as clinically-used antiviral agents against HIV. Among them, five nucleoside-type agents, i.e., 3'-azido-3'-deoxythymidine (AZT, generic name: Zidovudine), 2',3'-dideoxyinosine (ddI, generic name: Didanosine), 2,3'-dideoxycytidine (ddC, generic name: Zalcitabine), 2',3'-didehydro-3'-deoxythymidine (d4T, generic name: Stavudine), and 2'-deoxy-3'-thiacytidine (3TC, generic name: Lamivudine), and non-nucleoside-type agents, i.e., Nevirapine and Delavirdine, have been used as reverse transcriptase inhibitors. Four drugs have been recently introduced as protease inhibitors.

All of the nucleoside-type reverse transcriptase inhibitors are competitive inhibitors against the reverse transcriptase of HIV. Although they have excellent antiviral activity, they are strongly toxic to host cells, and their side effects have become problems. For example, severe anaemia and leukopenia due to bone marrow suppression have been reported as to AZT, and peripheral neuropathy and acute pancreatitis have been reported as to ddI, ddC, and d4T. In addition, it is also reported that resistant virus strains may appear when the nucleoside-type drugs are continuously administered alone for a prolonged period of time, which becomes a serious clinical problem. In particular, it has been recognized that 3TC rapidly loses its efficacy upon the proliferation of resistant viruses, and therefore, this drug is used in combination with AZT and its sole application has not been approved.

Recently, several compounds called "allosteric inhibitors" having a novel mode of action are reported (European Patent Publication Nos. 384522, 429987, 462800, 420763 and other); the inhibitors bind to reverse transcriptase of HIV at a site other than the substrate binding site to inhibit the function of the enzyme. These compounds have potent inhibitory activity against HIV, and in addition, they are highly specific to HIV and have extremely low cytotoxicity compared to the nucleoside-type drugs.

### Disclosure of the Invention

By using the nucleoside-type drugs and the allosteric inhibitor in combination, inhibitory activities of the two types of drugs with distinguishable mode of actions will effect synergistically on the enzyme, and as a consequence, their doses, side effects, and possibility of the appearance of resistant strains can possibly be lowered. The inventors of the present invention previously studied combined effects of various allosteric inhibitors and 2',3'-dideoxyribonucleosides, and found that potent synergistic effect was obtained by using 6-benzyl-1-ethoxymethyl-5-substituted uracil derivative as the allosteric inhibitor (Japanese Patent Unexamined Publication (KOKAI) No. (Hei) 7-97324/1995). As explained above, a combination therapy against HIV is significant because such therapy enhances efficacy and avoids the loss of efficacy due to the appearance of resistant viruses.

The inventor of the present invention further found that, as a combination of the 6-benzyl-1-ethoxymethyl-5-substituted uracil derivative and a nucleoside reverse transcriptase inhibitor, it is extremely advantageous to use three drugs in combination to suppress the appearance of resistant viral strains rather than to use the combination of two drugs disclosed in Japanese Patent Unexamined Publication (KOKAI) No. (Hei) 7-97324/1995. The present invention was achieved on the basis of this finding.

The present invention thus provides antiviral agents comprising as active ingredients a 6-benzyl-1-ethoxymethyl-5-substituted uracil derivative represented by the following general formula (I): 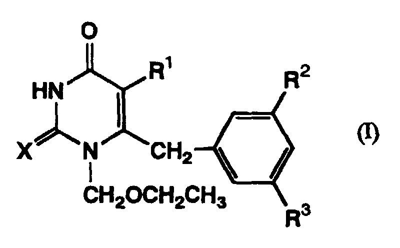 wherein X represents oxygen atom or sulfur atom, R¹ represents ethyl group or isopropyl group, and R³ and R³ independently represent hydrogen atom, a C₁-C₃ alkyl group, or a halogen atom, together with two or more substances selected from the group consisting of nucleoside-type reverse transcriptase inhibitors and esters thereof.

According to preferred embodiments of the present invention, there are provided: the aforementioned antiviral agents wherein the two or more substances selected from the group consisting of nucleoside-type reverse transcriptase inhibitors and esters thereof are two or more substances selected from the group consisting of the compound represented by Formula (II) [3'-azido-3'-deoxythymidine (AZT)], the compound represented by Formula (III) [2'-deoxy-3-thiacytidine (3TC)], the compound represented by Formula (IV) [PMEA], the compound represented by Formula (V) [PMPA], the compound represented by Formula (VI) [1592U89 succinate], the compound represented by Formula (VII) [2',3'-dideoxyinosine (ddI)], and the compound represented by Formula (VIII) [3'-deoxy-2',3'-didehydrothymidine (d4T)] and esters thereof such as phosphate esters thereof; 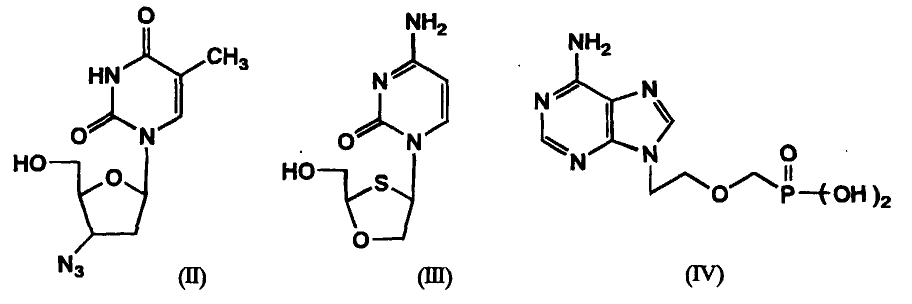 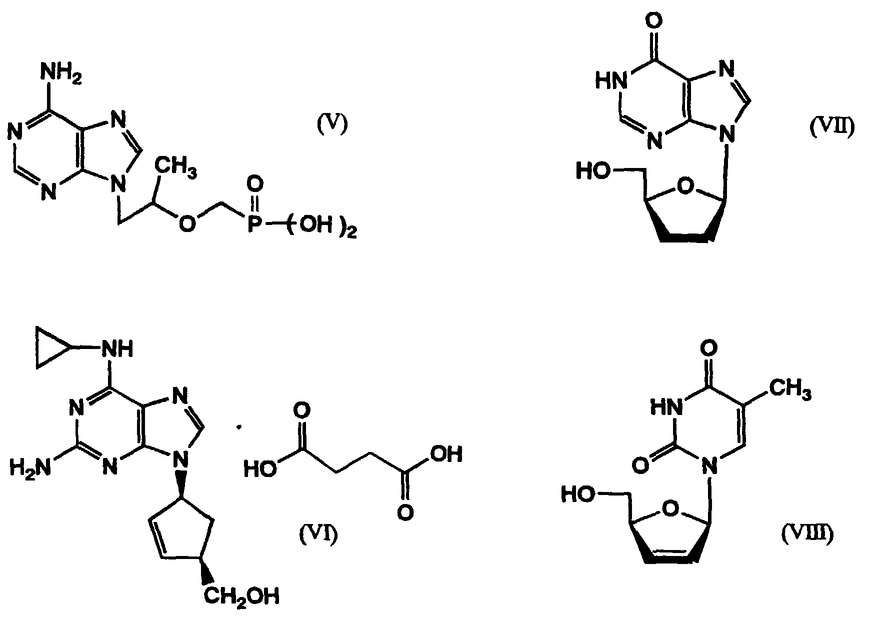 the aforementioned antiviral agents wherein the 6-benzyl-1-ethoxymethyl-5-substituted uracil derivative is 6-benzyl-1-ethoxymethyl-5-isopropyluracil; the aforementioned antiviral agents wherein two nucleoside-type reverse transcriptase inhibitors are selected from the group consisting of 3'-azido-3'-deoxythymidine, 2'-deoxy-3'-thiacytidine, 2',3'-dideoxyinosine, and 3'-deoxy-2',3'-didehydrothymidine; the aforementioned antiviral agents wherein the nucleoside-type reverse transcriptase inhibitors are the combination of 3'-azido-3'-deoxythymidine and 2'-deoxy-3'-thiacytidine; the aforementioned antiviral agents which are anti-retroviral agents; and the aforementioned antiviral agents which are anti-human immunodeficiency viral agents.

As a preferred embodiment of the aforementioned antiviral agent, there is provided the antiviral agents in the form of a pharmaceutical composition which comprises the aforementioned 6-benzyl-1-ethoxymethyl-5-substituted uracil derivative, two or more substances selected from the group consisting of nucleoside-type reverse transcriptase inhibitors and esters thereof such as phosphate esters thereof, and one or more pharmaceutically acceptable additives.

According to further aspects of the present invention, there are provided: a method for preparing the aforementioned antiviral agent; a method for therapeutic and/or preventive treatment of a viral infection which comprise a step of administering to a patient a therapeutically and/or preventively effective amount of the aforementioned 6-benzyl-1-ethoxymethyl-5-substituted uracil derivative represented by the above general formula (I), and two or more substances selected from the group consisting of nucleoside-type reverse transcriptase inhibitors and esters thereof such as phosphate esters thereof; and a use of the aforementioned 6-benzyl-1-ethoxymethyl-5-substituted uracil derivative represented by the above general formula (I) or a use of a substance selected from the group consisting of nucleoside-type reverse transcriptase inhibitors and esters thereof such as phosphate esters thereof for the manufacture of the antiviral agent comprising as active ingredients the 6-benzyl-1-ethoxymethyl-5-substituted uracil derivative represented by the above general formula (I) together with two or more substances selected from the group consisting of the nucleoside-type reverse transcriptase inhibitors or esters thereof such as phosphate esters thereof.

### Brief Explanation of the Drawing

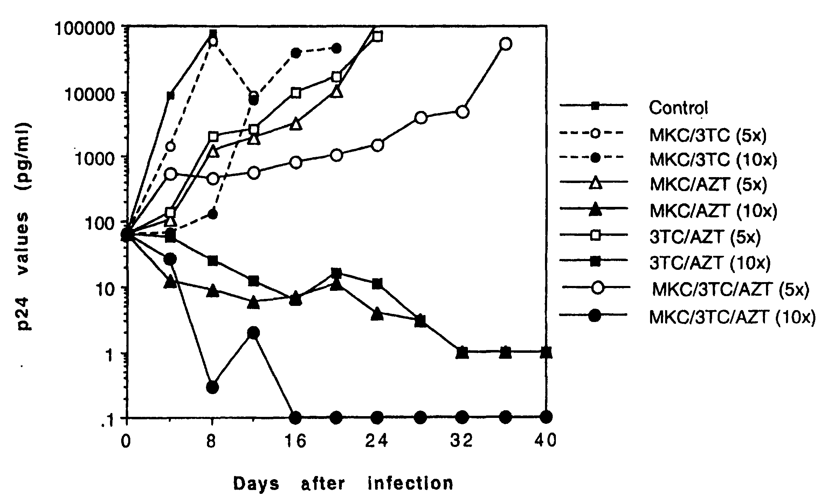

Fig. 1 shows results of the amount of viral antigen during subcultures of MT-4 cells infected with HIV for a long period of time in the presence of various combined drugs.

### Best Mode for Carrying Out the Invention

The antiviral agents of the present invention comprise, as active ingredients, a 6-benzyl-1-ethoxy methyl-5-substituted uracil derivative represented by the aforementioned general formula (I) together with two or more substances selected from the group consisting of nucleoside reverse transcriptase inhibitors and esters thereof such as phosphate esters thereof.

The 6-benzyl-1-ethoxymethyl-5-substituted uracil derivatives are disclosed in European Patent Publication No. 420763, and they can be prepared by the method disclosed in the document or a method similar to the disclosed method. Examples of the C₁-C₃ alkyl group defined by R² and R³ include, for example, methyl group, ethyl group, propyl group, and isopropyl group, and examples of the halogen atom include, for example, fluorine atom, chlorine atom, and bromine atom. For the antiviral agent of the present invention, compounds wherein X represents oxygen atom and R² and R³ represent hydrogen atoms are preferably used, and among them, compounds wherein R¹ represents isopropyl group are more preferably used.

Examples of the nucleoside-type reverse transcriptase inhibitors include 3'-azido-3'-deoxythymidine (AZT) represented by the aforementioned formula (II), 2'-deoxy-3'-thiacytidine (3TC) represented by the aforementioned formula (III), PMEA represented by the aforementioned formula (IV), PMPA represented by the aforementioned formula (V), 1592U89 succinate represented by the aforementioned formula (VI), 2',3'-dideoxyinosine (ddI) represented by the aforementioned formula (VII), and 3'-deoxy-2',3'-didehydrothymidine (d4T) represented by the aforementioned formula (VIII), and phosphate esters thereof, and physiologically hydorolyzable esters such as bis-Pom PMEA, bis-Poc PMPA and the like. However, any substances that are classified into the nucleoside-type reverse transcriptase inhibitors and esters thereof can be used, and the above specific examples are not construed as limiting.

The nucleoside-type reverse transcriptase inhibitors can be synthesized by, as to ATZ, the methods described in J. Org. Chem., 29, 2076-2078 (1964); J. Org. Chem., 32, 817-818 (1967), J. Am. Chem. Soc. 86, 3585-3586 (1964), Chemistry in Organic Synthesis (Yuhki Gohsei Kagaku), 48, 907-920 (1990), Chemistry in Organic Synthesis, 50, 535-544 (1992) and the like, or methods similar to the described methods. 3TC can be prepared by the methods described in J. Org. Chem, 57, 2217-2219 (1992), Tetrahedron Lett., 33, 4629-4632 (1992), WO91/11186 and the like, or methods similar to the described methods. PMEA and bis-POM PMEA can be prepared by the methods disclosed in Japanese Patent Unexamined Publication No. (Hei) 4-230694/1992 and the like, or methods similar to the disclosed methods. PMPA and bis-Poc PMPA can be prepared by the methods described in Collect. Czech. Chem. Commun., 60, 1196-1212 (1995) or methods similar to the described method. 1592U89 succinate can be prepared by the methods described in WO96/06844 and the like, or methods similar to the described methods. AZT and d4T can be purchased from Sigma Co. or the like and ddI from P.L. Biochmicals.

The antiviral agents of the present invention are applied as medicaments for preventive and/or therapeutic treatment of infections caused by various viruses. Examples of target viruses include, for example, retroviruses such as oncovirus, lenti virus, and spuma virus. HIV is particularly preferred as the target virus.

The antiviral agents of the present invention may be administered to a human orally, or parenterally such as by intravascular administration, enteral administration, and topical administration. Doses may be appropriately chosen depending on, for example, the age, conditions, or body weight of a patient. Generally, a dose of the 6-benzyl-1-ethoxymethyl-5-substituted uracil derivative represented by the aforementioned formula (I) as a sole administration may be 1-100 mg/kg body weight, preferably 5-50 mg /kg body weight per day. A dose of the nucleoside-type reverse transcriptase inhibitor or an ester thereof as a sole administration may be 1-100 mg/kg body weight preferably 5-50 mg /kg body weight per day. Accordingly, an amount of each of the above ingredients to be formulated may be appropriately chosen so as to be equal to or less than the aforementioned dose. The antiviral agent of the present invention may be administered once or more times a day.

A formulation ratio of the 6-benzyl-1-ethoxymethyl-5-substituted uracil derivative and the two or more substances selected from the group consisting of the nucleoside reverse transcriptase inhibitors and esters thereof may preferably be in a range of 100:1 to 1:100, and more preferably 30:1 to 1:30. A formulation ratio of each of the two or more substances selected from the group consisting of the nucleoside reverse transcriptase inhibitors and esters thereof may be chosen based on the efficacy of each substance.

As the method of the present invention for therapeutic and/or preventive treatment of viral infections, an example of applicable methods includes a method comprising the step of administration of the aforementioned antiviral agent according to the present invention. In addition, examples also include a method comprising the step of simultaneously or successively administering a medicament comprising the 6-benzyl-1-ethoxymethyl-5-substituted uracil derivative and a medicament comprising two or more substances selected from the group consisting of the nucleoside-type reverse transcriptase inhibitors and esters thereof; and a method comprising the step of simultaneously or successively administering a medicament comprising the 6-benzyl-1-ethoxymethyl-5-substituted uracil derivative, a medicament comprising a substance selected from the group consisting of the nucleoside-type reverse transcriptase inhibitors and esters thereof, and a medicament comprising a substance which is different from the above-mentioned substance and selected from the group consisting of the nucleoside-type reverse transcriptase inhibitors and esters thereof.

As the combinations of the 6-benzyl-1-ethoxymethyl-5-substituted uracil derivative and the two or more substances selected from the group consisting of the nucleoside-type reverse transcriptase inhibitors and esters thereof, examples include those set out below which may preferably be used for the antiviral agent and method for preparing thereof, and the method for therapeutic and/or preventive treatment of viral infections according to the present invention. However, combinations applicable in the present invention are not limited to those mentioned below.
6-benzyl-1-ethoxymethyl-5-substituted uracil derivative + AZT + 3TC;
6-benzyl-1-ethoxymethyl-5-substituted uracil derivative + AZT + PMEA or bis-Pom PMEA;
6-benzyl-1-ethoxymethyl-5-substituted uracil derivative + AZT + PMPA or bis-Poc PMPA;
6-benzyl-1-ethoxymethyl-5-substituted uracil derivative + AZT + 1592U89;
6-benzyl-1-ethoxymethyl-5-substituted uracil derivative + ddI + 3TC;
6-benzyl-1-ethoxymethyl-5-substituted uracil derivative + ddI + PMEA or bis-Pom PMEA;
6-benzyl-1-ethoxymethyl-5-substituted uracil derivative + ddI + PMPA or bis-Poc PMPA;
6-benzyl-1-ethoxymethyl-5-substituted uracil derivative + ddI +1592U89;
6-benzyl-1-ethoxymethyl-5-substituted uracil derivative + ddC +3TC;
6-benzyl-1-ethoxymethyl-5-substituted uracil derivative + ddC + PMEA or bis-Pom PMEA;
6-benzyl-1-ethoxymethyl-5-substituted uracil derivative + ddC + PMPA or bis-Poc PMPA;
6-benzyl-1-ethoxymethyl-5-substituted uracil derivative + ddC + 1592U89 succinate;
6-benzyl-1-ethoxymethyl-5-substituted uracil derivative + d4T + 3TC;
6-benzyl-1-ethoxymethyl-5-substituted uracil derivative + d4T + PMEA or bis-Pom PMEA;
6-benzyl-1-ethoxymethyl-5-substituted uracil derivative + d4T + ddI;
6-benzyl-1-ethoxymethyl-5-substituted uracil derivative + d4T + PMPA or bis-Poc PMPA;
6-benzyl-1-ethoxymethyl-5-substituted uracil derivative + d4T + 1592U89 succinate
6-benzyl-1-ethoxymethyl-5-substituted uracil derivative + 3TC + PMEA or bis-Pom PMEA;
6-benzyl-1-ethoxymethyl-5-substituted uracil derivative + 3TC + PMPA or bis-Poc PMPA;
6-benzyl-1-ethoxymethyl-5-substituted uracil derivative + 3TC + 1592U89 succinate;
6-benzyl-1-ethoxymethyl-5-substituted uracil derivative + AZT + 3TC+ PMEA or bis-Pom PMEA;
6-benzyl-1-ethoxymethyl-5-substituted uracil derivative + AZT + 3TC+ PMPA or bis-Poc PMPA; and
6-benzyl-1-ethoxymethyl-5-substituted uracil derivative + AZT + 3TC + 1592U89 succinate.

The antiviral agent of the present invention may preferably be provided in the form of a pharmaceutical composition which comprises the aforementioned active ingredients together with one or more pharmaceutically acceptable additives such as ordinarily used pharmaceutical carriers and excipients. A pharmaceutical preparation suitable for oral or parenteral administration may appropriately be chosen by one of ordinary skilled in the art. Carriers used for the manufacture of the antiviral agent of the present invention may be solid or liquid. Examples of the solid carriers include, for example, lactose, china clay (kaolin), sucrose, crystalline cellulose, corn starch, talc, agar, pectin, stearic acid, magnesium stearate, lecithin, and sodium chloride. Examples of the liquid carrier include, for example, glycerin, peanut oil, polyvinylpyrrolidone, olive oil, ethanol, benzyl alcohol, propylene glycol, physiological saline, and water.

The form of pharmaceutical formulations of the antiviral agent of the present invention are not particularly limited. Examples of the pharmaceutical formulations include, for example, tablet, powder, granule, capsule, suppository, and troche where one or more solid carriers are used. Examples of the drug formulation also include, for example, syrup, emulsion, soft gelatin capsule, cream, gel, paste, spray, injection, and drip infusion where one or more liquid carriers are used.

### Examples

The present invention will be explained more specifically with reference to examples. However, the scope of the present invention is not limited to the examples set out below.

### Reference Example 1: Synthesis of 6-benzyl-1-ethoxymethyl-5-isopropyluracil (abbreviated as "MKC-442" hereinafter)

5-Isopropyluracil (18 g) was suspended in dichloromethane (230 ml), and the suspension was added with bistrimethylsilylacetamide (60.7 ml), and then the mixture was stirred at room temperature for one hour. The resulting solution was added with tetra-n-butylammonium iodide (0.46 g) and chloromethyl ethyl ether (10.7 ml) at room temperature, and the mixture was stirred for 1.5 hours. Cold water (50 ml) was added to the mixture, and then washing and separation were repeated twice. The organic layer was concentrated under reduced pressure, and the residue was added with h-heptane (100 ml) and crystallized at room temperature to obtain 1-ethoxymethyl-5-isopropyluracil (22.2 g). Melting point: 78.4°C.

A 1N solution of lithium diisopropylamide in tetrahydrofuran (357 ml) was cooled to -10 to -15°C under nitrogen flow, and then added dropwise with a solution of 1-ethoxymethyl-5-isopropyluracil (22.2 g) in tetrahydrofuran (100 ml) at a rate that maintained an internal temperature at -10 to -15°C. After the dropwise addition, the mixture was stirred for 1 hour at -10 to -15°C. Then, benzaldehyde was added dropwise to the mixture at a rate that maintained an internal temperature at -10 to -15°C. The mixture was stirred at -10 to -15°C for one hour. The reaction was stopped by the addition of acetic acid (40.8 ml), and then the reaction mixture was warmed up to room temperature and added with half-saturated brine (100 ml) to carry out washing and separation. The organic layer was added with hexane (220 ml), and extracted twice with 1N solution of sodium hydroxide (150 ml). The aqueous layer was neutralized with hydrochloric acid, and extracted with toluene (200 ml). After the toluene was removed under reduced pressure, the residue was dissolved in pyridine (100 ml), and the solution was added with acetic anhydride (270 g) and left at room temperature for 16 hours. Then, cold water (100 ml) was added to the mixture, and the mixture was left at room temperature for 16 hours. Aqueous pyridine was removed under reduced pressure, and the resulting crystals were collected by filtration to obtain 6-(α-acetoxybenzyl)-1-ethoxymethyl-5-isopropyluracil (13.3 g). Melting point: 158°C.

The 6-(α-acetoxybenzyl)-1-ethoxymethyl-5-isopropyluracil (13.3 g) was dissolved in dioxane (53 ml), and the solution was added with 5% palladium/carbon (0.66 g), and then the mixture was stirred under hydrogen atmosphere at atmospheric pressure at 50°C for four hours. After palladium/carbon was removed, dioxane was evaporated under reduced pressure, and the residue was added with ethanol (50 ml) and crystallized in a refrigerator to give 6-benzyl-1-ethoxymethyl-5-isopropyluracil (MKC-442, 10g). Melting point: 109-110°C.

### Reference Example 2: Nucleoside reverse transcriptase inhibitors

3TC: BHC-189 in (±)-cis-configulation was synthesized according to the method described in WO 91/11186. The product was subjected to optical resolution by using a column for optical resolution (AS, Daicell) and a mixture of hexane and ethanol (6:4) as a developing solvent to obtain 3TC having a negative optical rotation with a shorter retention time. AZT was purchased from Sigma to use in the experiments below.

### Example 1: Inhibitory effect on the appearance of drug resistant strains in long-term cultivation of HIV-1 infected cells

For therapeutic treatment of a patient with HIV infection, an antiviral agent is administered for a prolonged period. However, it has been recognized that drug resistant viruses may appear under the treatment using a single drug, and as a consequence, the viruses may proliferate again. To avoid this phenomenon and maintain efficacy of a drug for a long period of time, combined therapy has recently been studied; for example, combinations of two nucleoside-type reverse transcriptase inhibitors, as well as combinations of a nucleoside-type reverse transcriptase inhibitor and a protease inhibitor are practically used. The inventor of the present invention found that the efficacy of a 6-benzyl-1-ethoxymethyl-5-substituted uracil derivative was synergistically enhanced when used in combination with a nucleoside-type reverse transcriptase inhibitor, and that the combined therapy using the two drugs was superior to therapies using a single drug. In this example, comparisons were made between the use of a combination of three medicaments in total, i.e., MKC-442 together with two nucleoside-type reverse transcriptase inhibitors, and combined uses of two medicaments chosen from these medicaments. The experiments were carried out to compare period of time in which the drug efficacy was maintained in a long-term cultivation of HIV-1 infected cells.

MT-4 cells (human T-cell clone which is killed by HIV infection), cultured in RPMI 1640 medium supplemented with 10% heat-treated fetal bovine serum, 100 U/ml of penicillin G, and 100 µg/ml of streptomycin, were infected with HIV-1 at a multiplicity of infection of 0.1, and after two hours, the cells were washed with the culture medium. The infected cells were inoculated to each well of 24-well plate in a volume of 100 µl per well at a concentration of 1×10⁵/ml. The cells were added with a given volume of a mixture of drugs dissolved in dimethyl sulfoxide, and then the cells were incubated at 37°C. The content ratios of the drugs were decided based on 50% inhibitory concentrations (IC₅₀) of the respective drugs which had been determined in separate experiments by using the respective drugs alone. The content ratio of MKC-442, 3TC, and AZT was 10:100:1. Furthermore, an IC₅₀ value was measured in a separate experiment by using a mixed solution prepared so as to contain the drugs at the aforementioned content ratio. A solution containing the mixture of the drugs at a five-fold or ten-fold concentration of the IC₅₀ value was practically used for the long-term cultivation. For the combination of the two drugs, total concentrations of MKC-442/3TC (1/10) were 0.6 and 1.2 µM, those of MKC-442/AZT (10/1) were 0.075 and 0.15 µM, and those of 3TC/AZT (100/1) were 1.1 and 2.2 µM. For the combination of three drugs, concentrations of MKC-442/3TC/AZT (10/100/1) were 0.5 and 1.0 µM. Four days after the start of the cultivation, the cells were subcultured by using a fresh medium containing the same amounts of the drugs, and subcultures were repeated every 4 days. When the cells were totally killed by the proliferation of the viruses, the cultivation was terminated. Cell supernatant was collected during the subculture, and the amount of HIV p24 antigen was measured by using an assay kit.

All of the cells were killed by HIV after twice subcultures in the absence of the drugs. Among the combinations of the two drugs, MKC-442/3TC exhibited weak inhibitory activity against virus proliferation at the five-fold concentration of the IC₅₀ value, however, the cells survived in the fifth subculture (20 days) at the ten-fold concentration. As to the combination of MKC-442/AZT or 3TC/AZT, the viral proliferation was not ceased at the five-fold concentration, however, the cells survived for 24 days in each of the cultures. At the ten-fold concentration, the amount of the virus in the medium was gradually decreased, and apparently higher inhibitory effect on proliferation was observed than the result obtained by the five-fold concentration. Viral proliferation was more strongly inhibited by the combination of three drugs than the combinations of two drugs, i.e., the cells survived for 36 days at the five-fold concentration. In particular, at the ten-fold concentration, the amount of the virus was lowered below a detection limit on the 16th day. Genomic analysis by PCR verified that HIV was completely eliminated from the culture system. From these results, it can be clearly understood that the combined therapy using the three drugs is extremely advantageous therapeutic treatment compared to the therapies using two drugs in combination to suppress the appearance of drug resistant viruses and to maintain efficacy of the drugs for a long period of time.

### Industrial Applicability

The antiviral agents of the present invention comprising three or more active ingredients can exhibit synergistic antiviral activity. Accordingly, the agent can reduce respective doses of drugs, and are expected to suppress the appearance of drug-resistant viruses for a long period of time.

## Claims

1. An antiviral agent comprising as active ingredients a 6-benzyl-1-ethoxy-methyl-5-substituted uracil derivative represented by the following general formula (I): 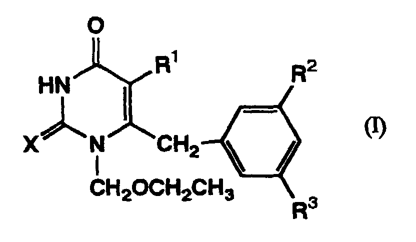 wherein X represents oxygen atom or sulfur atom, R¹ represents ethyl group or isopropyl group, and R² and R³ independently represent hydrogen atom, a C₁-C₃ alkyl group, or a halogen atom, together with two or more substances selected from the group consisting of nucleoside-type reverse transcriptase inhibitors and esters thereof.

2. The antiviral agent according to claim 1, wherein the two or more substances selected from the group consisting of the nucleoside-type reverse transcriptase inhibitors and esters thereof are two or more substances selected from the group consisting of the compound represented by Formula (II), the compound represented by Formula (III), the compound represented by Formula (IV), the compound represented by Formula (V), the compound represented by Formula (VI), the compound represented by Formula (VII), and the compound represented by Formula (VIII), and esters thereof.

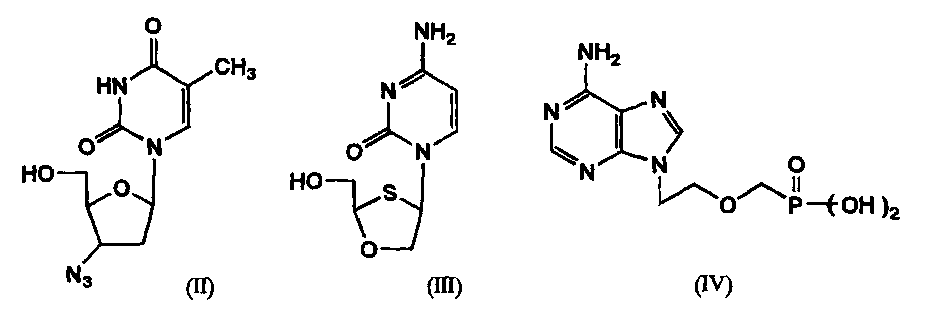

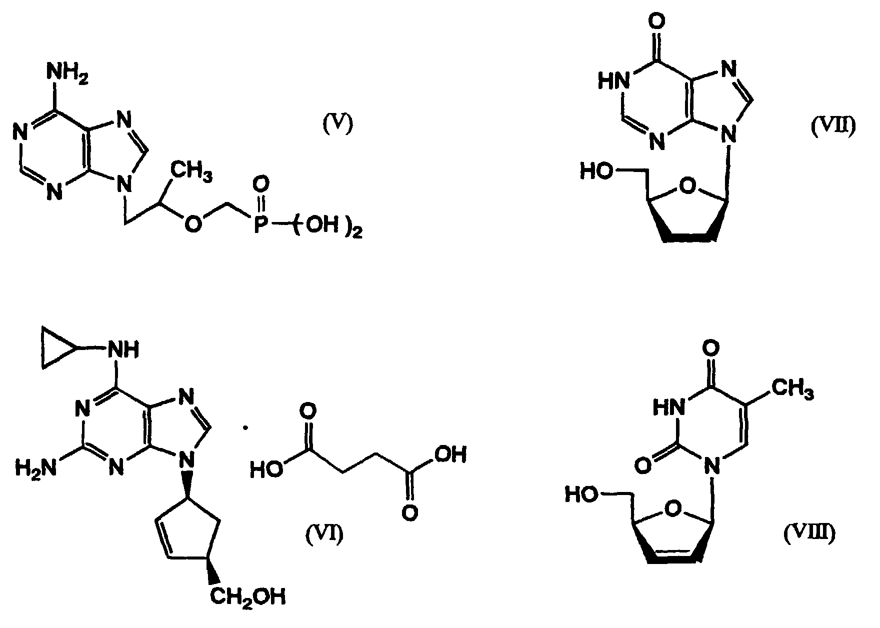

3. The antiviral agent according to claim 1 or claim 2, wherein the 6-benzyl-1-ethoxymethyl-5-substituted uracil derivative is 6-benzyl-1-ethoxymethyl-5-isopropyluracil.

4. The antiviral agent according to any one of claims 1 to 3, wherein the two nucleoside-type reverse transcriptase inhibitors are selected from the group consisting of 3'-azido-3'-deoxythymidine, 2'-deoxy-3'-thiacytidine, 2',3'-dideoxyinosine, and 3'-deoxy-2',3'-didehydrothymidine.

5. The antiviral agent according to any one of claims 1 to 3, wherein the two nucleoside-type reverse transcriptase inhibitors are the combination of 3'-azido-3'-deoxythymidine and 2'-deoxy-3'-thiacytidine.

6. The antiviral agent according to any one of claims 1 to 5 which is an anti-retroviral agent.

7. The antiviral agent according to any one of claim 1 to 6 which is anti-human immunodeficiency viral agent.

8. The antiviral agent according to any one of claim 1 to 7, wherein said agent is in the form of a pharmaceutical composition which comprises the 6-benzyl-1-ethoxymethyl-5-substituted uracil derivative, two or more substances selected from the group consisting of the nucleoside-type reverse transcriptase inhibitors and esters thereof, and one or more pharmaceutically acceptable additives.

9. A method for therapeutic and/or preventive treatment of a viral infection, which comprises the step of administering to a patient a therapeutically and/or preventively effective amount of the 6-benzyl-1-ethoxymethyl-5-substituted uracil derivative represented by the general formula (I) defined in claim 1, and two or more substances selected from the group consisting of the nucleoside-type reverse transcriptase inhibitors and esters thereof
